# EUROPEAN PATENT APPLICATION

(11) **EP 3 278 724 A1**
(43) Date of publication of application: **07.02.2018**
(21) Application number: 16742726.9
(22) Date of filing: 25.01.2016
(51) Int. Cl.: A61B 5/0402, A61B 5/0476, A61B 5/0205

(54) **WEARABLE ELECTROCARDIOGRAPHIC DETECTION DEVICE AND WEARABLE PHYSIOLOGICAL DETECTION DEVICE**

(30) Priority: 26.01.2015 CN 201510038000; 26.01.2015 CN 201510038236; 26.01.2015 CN 201510037872; 22.01.2016 CN 201620064281 U; 22.01.2016 CN 201620064551 U; 22.01.2016 CN 201620064148 U; 22.01.2016 CN 201610043828
(71) Applicant: Chou, Chang-An, 1070 Wien (AT)
(72) Inventor: Chou, Chang-An, 1070 Wien (AT)
(86) International application number: PCT/CN2016/072022
(87) International publication number: WO 2016/119664

(57) **Abstract**

The present invention discloses wearable electrocardiographic measurement device which includes a first electrode and a second electrode, wherein the first electrode is mounted on a user's body via a wearable structure, and the second electrode is implemented to contact the user's upper limb, neck or shoulder, so as to achieve a loop for acquiring electrocardiographic signals.

## Description

### FIELD OF THE INVENTION

The present invention is related to a wearable electrocardiographic measurement device, and more particularly, related to a wearable electrocardiographic measurement device which includes a structure for providing an active force to force electrode(s) to contact the skin, so as to improve the quality of acquired physiological signals.

### BACKGROUND OF THE INVENTION

It is known that electrocardiographic measurement devices (ECG devices) can be used to exam many kinds of heart-related diseases, such as, arrhythmia, hypertrophic cardiomyopathy caused from hypertension or valvular heart diseases, myocardial infarction, or ischemic heart disease.

When people have heart problem and go to hospital for examination, the traditional way is to use the 12-lead ECG device which can detect the heart problems more comprehensively. However, if the heart problem is occasional, e.g., arrhythmia, there has high possibility that the occasional heart syndrome does not happen and the examination cannot detect it. Therefore, for solving this problem, Holter monitor, which can be worn for long term, e.g., 24 hours to several days, is used to detect and record the occasional heart syndrome. Similarly, an ECG event recorder also can be worn for long term, but differently, the ECG event recorder is activated by the user, for example, when the user feels the heart problem, he/she can press the button to start the recording of electrocardiogram for a short term, e.g., 30 seconds prior and 30 second post the button being pressed, namely, although the device will detect the user's electrocardiogram, it only records after the button is pressed. Besides, Holter monitor is usually also used to monitor the heart condition after surgery or taking medicine, so as to confirm the treatment effect.

No matter Holter monitor or ECG event recorder, the setting thereof includes multiple ECG electrodes, which are connected to a main machine via multiple cables, so that the user has to have all those electrodes attached on the body and carry the main machine during the whole monitoring period. Therefore, not only the user will feel inconvenient, but it might also cause skin allergies due to the long-term attachment of electrodes. Both are reasons that make the user step back. And, sometimes, it is also possible that even after the long-term monitoring, the recorded electrocardiogram does not include any occasional syndrome. Further, this kind of examination should be settled by the medical personnel, for example, the attachment of electrodes should be completed in the hospital. In addition, after the long-term recording, it will need to wait the doctor to interpret and analyze the electrocardiogram, so that when the user knows the examination result, it will be at least few days later. Therefore, it not only is complicated, but also lacks of promptness.

Therefore, for solving these drawbacks, a handheld ECG device is proposed, which doesn't need to attach the electrodes on the user's body, so that the inconvenience of long-term electrode attachments and the complexity of wirings can be eliminated. For example, US7149571 and US7197351 disclosed a handheld ECG device having dry electrodes mounted on the surface thereof, and when there is a need, the user can perform the ECG measurement by contacting the electrodes with hands and/or other body portion at any time. This provides the flexibility. Moreover, this kind of ECG device usually is equipped with analysis algorithm and display, so that the user can see the results immediately, and no need to go to the hospital. Consequently, it is suitable for using at home. And, for users who highly care about the heart condition, this provides an easy and convenient way.

Then, the portable electronic devices, e.g., smart phones, become more and more popular, so that recently, the new type ECG device is to combine with the portable electronic device. For example, US8615290 disclosed a device similar to the handheld ECG device with dry electrodes, and the only difference is it employs the operation interface of mobile phone to control the operation of the device. This can reduce the number of portable devices the user carried every day.

However, although the devices described above provide the convenience of portability, the volume thereof is still too big since the shape thereof has to fit the ergonomics of the holding hand and the display for showing will occupy a certain space. Besides, since the electrodes are not attached on the body surface all the time, when there is the need to record the electrocardiogram, it will take more steps to complete the measurement, for example, the user has to first take out the device and power on the device, and then he/she can start the recording. Therefore, there is high possibility that the user might miss the recording chance.

Furthermore, when it needs two hands to operate, the instability might occur, e.g., hand shaking, so as to reduce the quality of electrocardiogram, e.g., baseline drift, or waveform deformation, and thus influence the analysis. Moreover, the hand operation also might be easily influenced by electromyographic signals which are arisen from the muscle tension, for example, if the user wants to remain the stability of hand, the muscle tension might rise, or if the user intentionally wants to ensure the contact between the electrode and the skin, the muscle tension also will rise.

Consequently, there is a need to have a wearable electrocardiographic measurement device which not only can provide the user a convenient operation manner, but also can reduce all above possible influences.

When the ECG device can be worn on the user's body, the electrocardiogram can be used to obtain other physiological information, for example, the time sequence of heart beats from the electrocardiogram can be the basis of HRV (Heart Rate Variability) analysis, and thus to obtain the information about ANS (Autonomic nervous system) activity, and in accordance with these information, it will be able to guide the user to perform breath training which is useful in improving ANS balance.

Because one of the main causes of arrhythmia is ANS disorders, when the user wears the ECG device for recording the electrocardiogram of arrhythmia in real time, if the same device can simultaneously provide the function to improve arrhythmia, for the user, it will be a more complete solution.

### SUMMARY OF THE INVENTION

The object of the present invention is to provide a wearable electrocardiographic measurement device, whose electrode for acquiring electrocardiographic signals is implemented to be wearable type, such that the contact between the electrode and the skin can be achieved without user's exerting force.

Another object of the present invention is to provide a wearable electrocardiographic measurement device which is mounted on a finger of the user through a finger-worn structure, and the contact of electrode thereof with skin of the finger is achieved while the wearing of the device on the finger is completed.

Another object of the present invention is to provide a wearable electrocardiographic measurement device which is mounted on an ear of the user through an ear-worn structure, and the contact of electrode thereof with skin of the ear or around the ear is achieved while the wearing of the device on the ear is completed.

Another object of the present invention is to provide a wearable electrocardiographic measurement device which employs a finger-worn structure and an ear-worn structure to respectively carry two electrodes and mount thereof on a finger and an ear of the user, so as to minimize the interference from electromyographic signals, and further provide an way to continuously and long term acquire electrocardiographic signals.

Another object of the present invention is to provide a wearable electrocardiographic measurement device which is mounted on a wrist of the user through a wrist-worn structure, and the contact of electrode thereof with skin around the wrist is achieved while the wearing of the device on the wrist is completed.

Still another object of the present invention is to provide a wearable physiological signal monitoring device which provides both functions of electrocardiographic signal measurement and electroencephalographic signal measurement and is mounted on the head of the user through a head-worn structure, such that contact of electrode thereof with skin of the head is achieved while the wearing of the device on the head is completed.

Further another object of the present invention is to provide a wearable electrocardiographic measurement device with two operation modes, for providing electrocardiograms of different heart angles and also for providing user the possibility to select operation mode based on the environment and operation habit.

Further another object of the present invention is to provide a wearable electrocardiographic measurement device which provides HRV analysis results of heart beat sequence, for revealing ANS activities of the user.

Further another object of the present invention is to provide a wearable electrocardiographic measurement device which is configured to obtain RSA information from the heart beat sequence, so as to be used as a basis for performing breathing training, thereby achieving the effect of influencing autonomous nervous system.

Further another object of the present invention is to provide a wearable physiological signal monitoring device which is configured to obtain respiratory pattern related information from the heart beat sequence, for performing a synchronization analysis among respiration, heart rate and electroencephalographic signals, so as to provide more information.

### BRIEF DESCRIPTION OF THE DRAWINGS

Figs. 1A-1B show finger-worn electrocardiographic measurement devices of preferred embodiments according to the present invention;
Figs. 2A-2C illustrate the operations of finger-worn electrocardiographic measurement device according to the present invention;
Fig. 3 shows the contact positions of electrode for the standard 12-lead electrocardiogram measurement;
Figs. 4A-4G show finger-worn electrocardiographic measurement devices of preferred embodiments according to the present invention
Fig. 5A shows an ear-worn electrocardiographic measurement device in a preferred embodiment according to the present invention;
Fig. 5B shows the operation of the ear-worn electrocardiographic measurement device according to the present invention;
Figs. 5C-5D show ear-worn electrocardiographic measurement devices of preferred embodiments according to the present invention;
Figs. 6A-6C show the electrode arrangements of the ear-worn electrocardiographic measurement device of preferred embodiments according to the present invention;
Fig. 7A show the area near the ear which is capable of being contacted by the ear-worn electrocardiographic measurement device according to the present invention;
Fig. 7B shows the physiological structure of the auricle;
Figs. 8A-8B show wearable electrocardiographic measurement devices of the present invention which employ ear-worn structure and ear-worn structure at the same time;
Figs. 9A-9B show wrist-worn electrocardiographic measurement devices of preferred embodiments according to the present invention;
Figs. 9C-9D show the operations of the wrist-worn electrocardiographic measurement device according to the present invention;
Figs. 9E-9F show the wearable electrocardiographic measurement devices of the present invention which employ wrist-worn structure and finger-worn structure at the same time;
Figs. 10A-10D show the wrist-worn electrocardiographic measurement devices according to the present invention which connect to an external electrode via a connection port;
Fig. 11A show the finger-worn electrocardiographic measurement device according to the present invention which connects to another finger-worn electrode via the connection port;
Fig. 11B show the ear-worn electrocardiographic measurement device according to the present invention which connects to a finger-worn electrode via the connection port;
Fig. 11C show the finger-worn electrocardiographic measurement device according to the present invention which connects to an ear-worn electrode via the connection port;
Figs. 12A-12B show the operations of ear-worn electrocardiographic measurement device according to the present invention which can acquire electrocardiogram via two acquisition loops;
Fig. 13 shows the operation of a head-worn electrocardiographic measurement device in a preferred embodiment according to the present invention; and
Figs. 14A-14C show the operations of glasses type electrocardiographic measurement devices of preferred embodiments according to the present invention.

### DETAILED DESCRIPTION OF THE INVENTION

The wearable electrocardiographic (ECG) measurement device according to the present invention includes a circuitry, a wearable structure, a first electrode and a second electrode, and an information providing unit, wherein the circuitry includes a processor for controlling the operation of the device, e.g., to acquire electrocardiograms via the first electrode and the second electrode, and the wearable structure is used to mount the device on a user's body while performing ECG measurement, so as to provide convenience. As to the information providing unit, it is used to provide information to the user, such as, operation information, physiological information, and analysis results.

The circuitry can be accommodated in the wearable structure, or alternatively, the device according to the present invention can further include a housing, so that the circuitry can be accommodated in the housing and/or the wearable structure. There is no limitation. Further, the material of the housing can be implemented to be identical to or different from that of the wearable structure, for example, if the same material is employed, two can be formed integrally, and oppositely, the materials can be changed to respectively suit the mounting positions of the housing and the wearable structure.

Furthermore, because the ECG measurement device of the present invention is implemented to be wearable type, the method of the information providing unit to provide information can have more possibilities, including but not limited, the information can be provided in a visual, auditory and/or tactile manner. For example, the information providing unit can be implemented as display element and/or LED (light emitting diode) element, so as to provide information by texts, figures, and/or lights; or the information providing unit also can be implemented as audio module, so as to provide information by the variation of sound frequency or volume or by voice; or the information providing unit further can be implemented as vibration element, so as to provide information by changing the vibration strength or the vibration duration.

Besides, the information providing unit can be configured to output the information to an external device via a wired transmission module or a wireless transmission module, so as to employ the external device to provide the user the information. Here, the external device can be, but not limited, a personal computer, a smart phone, a tablet, a smart watch, or any device which can be used to provide the information to the user, without limitation.

In the wearable ECG measurement of the present invention, particularly, the first electrode is configured to locate on a surface of the device which will contact the skin of the user while the user wears the wearable structure. Namely, the contact between the first electrode and the skin is achieved by the action of mounting the wearable structure on the user's body, so that there is no need for the user to apply force on the first electrode. Therefore, the electromyogram (EMG) raised by the muscle tension can be significantly reduced, thereby facilitating a good signal quality of the acquired ECG signals.

As to the second electrode, there have several possibilities. For example, the second electrode can be implemented to at least locate on another surface of the device for being contacted by another body portion of the user, e.g., finger, chest, namely, the second electrode should be exposed for being contacted while the first electrode is contacted with the skin through wearing the wearable structure. Here, it should be notice that the surfaces for mounting the first electrode and the second electrode can be on the wearable structure, the housing or any position of the device, without limitation. It only needs to pay attention that the first electrode and the second electrode should contact different body portions. The material of the electrodes can be metal, conductive rubber, or any conductive material. Further, electrodes also can be implemented as contactless electrodes, such as, capacitive electrodes, inductive electrodes, or electromagnetic electrodes, so as to increase the convenience.

Alternatively, the second electrode also can be mounted on the user's body through another wearable structure, and thus, both electrodes can be contact with the skin via the active forcing from the wearable structures.

Accordingly, in practice, first, through the wearable structure, the user can put the wearable ECG measurement device on the body thereof, e.g., the finer, the wrist, the ear or the head, and thus, the contact between the first electrode and the skin is already be achieved. Then, when there comes the need to detect the electrocardiogram, the user only needs to contact the second electrode with another skin portion, so that the loop for acquiring ECG signals can be achieved. Through this design, the user can conveniently and easily perform the ECG measurement at any time.

In the other way, when the second electrode is also mounted on the user's body through another wearable structure, the installation of electrodes for measurement will be completed just after the user puts on two wearable structures. Therefore, when the user wants to record the electrocardiogram, he/she only needs to, for example, press the start button, to start the acquisition, e.g., for 30 seconds or 1 minute. Alternatively, it also can be implemented to detect/record/analyze electrocardiograms just after the device is worn by the user, so that even a sudden heart condition happens, it still won't be missed. Hence, there is no limitation and can be selected to suit different situations.

Here, identically, the another wearable structure also can be configured to include a housing, and the second electrode also can be configured to locate on a surface of the wearable structure or the housing where can contact the skin as the another wearable structure is worn by the user. There is no limitation.

Because the ECG measurement device of the present invention is implemented as wearable type, except on the general method to start the ECG measurement, such as press the button, it also can employ other methods. For example, it can be implemented to have a switch near the second electrode which can be triggered by the contact of the second electrode with the skin, or alternatively, it also can be implemented to connect the second electrode with a physical state detecting unit, for sensing a physical state change of the electrode as contacting the skin, and thus, through checking the physical state change, the contact stability between the electrode and the skin can be determined, so as to decide if the ECG measurement can be started. Besides, the first electrode also can be connected with a physical state detecting unit without limitation.

Here, the physical state change includes, but not limited, pressure change and impedance change. For example, the physical state detecting unit can include a pressure sensing module for detecting the pressure change on the electrode, so as to determine if the force applied on the electrode is enough. Alternatively, the physical state detecting unit can be implemented as a switch which can sense the degree of pressure. Alternatively, the physical state detecting unit also can include an impedance sensing circuit or capacitance sensing circuit, for detecting the change of impedance or capacitance of the electrode. Thus, there is not limitation.

When making the decision, if the switch is not switched, and/or the physical state change is not conformed to a preset criteria, e.g., not higher than a threshold, which means the contact between the second electrode and the skin is not enough to perform the ECG measurement, so that the device will remain in an OFF state. On the other hand, if the switch has switched and/or the physical state change has reached the threshold, which means the contact is sufficient to perform the ECG measurement, and thus, the device will be converted into an ON state.

Here, particularly, whether the switch is switched or not or whether the physical state change has reached the preset criteria or not also can be used to determine the availability of the electrode, such as, to determine if electrode should be conducted. Namely, the electrode can be at first in a unavailable state until the switch has switched or the physical state change conformed to the preset criteria, and then, the electrode becomes available, e.g., be conducted. As a result, the signal quality of acquired electrocardiogram can be guaranteed, so as to facilitate a more accurate analysis result.

Furthermore, after the above determination, how to start the device and/or the measurement also has many choices. For example, in a preferred embodiment, the device is configured to automatically start the ECG measurement after a preset time, e.g., 3 seconds; in another preferred embodiment, the device is configured to convert into the state capable of performing ECG measurement after a preset time, e.g., 3 seconds, and if the available state remains, then the ECG measurement starts. Therefore, there are many kinds of possibilities, and can be changed based on the real situation without limitation.

Besides, with the above-mentioned determinations, the device of the present invention also can be configured to under a state of keep acquiring ECG signals, and only when an ECG characteristic, e.g., arrhythmia, is detected, the electrocardiogram is recorded, or the sampling rate or the amplification factor is adjusted, so as to completely record all possible ECG events.

Followings are the preferable illustrations of the wearable ECG measurement device of the present invention.

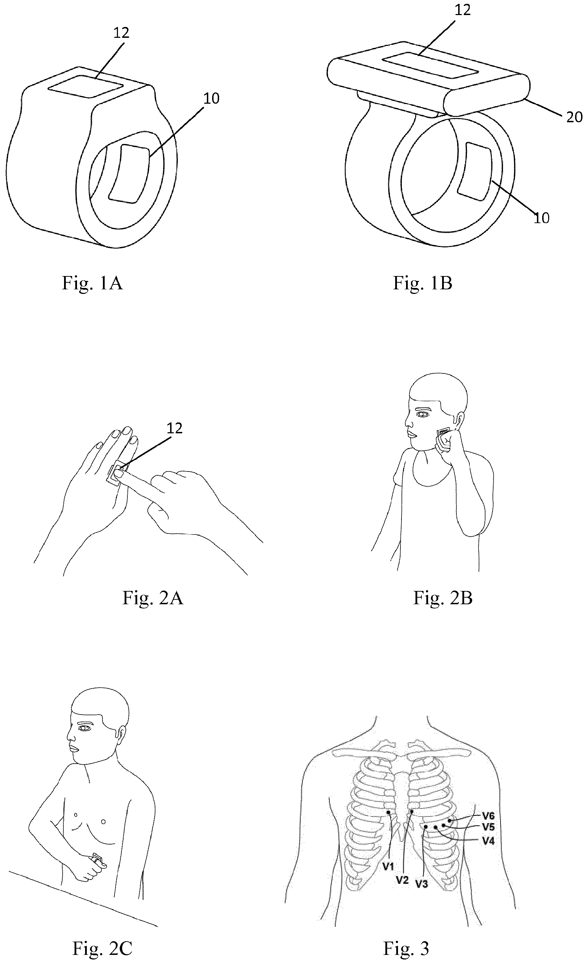

First, in a first aspect of the present invention, the wearable structure is implemented to be a finger-worn structure. Accordingly, the wearable ECG measurement device is carried by the finger-worn structure and thus mounted on a finger of a user. As shown in Fig. 1A, the finger-worn structure can be configured to contain the circuitry, or as shown in Fig. 1B, a housing 20 can be additionally combined with the finger-worn structure, and the circuitry can be accommodated in the housing and/or the finger-worn structure. There is no limitation.

The first electrode is configured to locate on a surface of the device where will contact the skin of the finger while the user wears the finger-worn structure. The second electrode is configured to at least locate on another surface of the device where will not contact the skin of the finger.

The reason to choose finger as the position for mounting the ECG measurement device is that, for general user, just like wearing a ring, the finger-worn structure is a familiar style without re-learning. The user only needs to put on the finger-worn structure and the contact between the first electrode and the skin can be completed. Then, when there comes the need to record the electrocardiograms, the user only needs to further contact the second electrode with a skin portion other than the limb of the finger, and the electrocardiogram acquisition can be achieved. The whole procedure and operation are simple, natural and convenient. Further, by utilizing the finger-worn structure to apply force on the first electrode for ensuring the contact with the skin, the force application by the user can be eliminated, and thus, the influence of muscle tension also can be significantly reduced.

In practice, there have many possible operation methods. For example, it can be implemented as the other hand touches the second electrode, as shown in Fig. 2A, or it also can be implemented as the user moves the finger wearing the device to touch another skin portion, such as, the cheek, as shown in Fig. 2B, and the torso, as shown in Fig. 2C. Therefore, there is no limitation.

Here, particularly, just because it is implemented as a finger-worn device and the finger can reach almost any portion of human body, the user can freely move the finger wearing the device to contact any skin portion other than the limb with almost no limitation, so that the operation possibilities are significantly increased. The user can select to contact a desired position in accordance with the operation environment and the real demand, which provides the convenience.

Consequently, through this concept, the user can easily obtain electrocardiograms at different angles, so as to more precisely evaluate the heart condition. Fig. 3 shows the contact positions for obtaining the standard 12-Leads electrocardiogram. Through the finger-worn ECG measurement device, conveniently, the user can wear the device first and then contact the second electrode with points V1∼V6 without difficulty, thereby obtaining the electrocardiograms at different angles.

When performing the ECG measurement, it is known that every two electrodes can obtain an electrocardiogram at one angle, namely, the positions of electrodes decide the angle of electrocardiogram of heart activity. Heart is a three-dimensional organ, and a lesion might be located at any portion of the heart. For example, the examination of myocardial infarction has to check the ST shift in the electrocardiogram which is caused from the damaged myocardium. However, if the damaged myocardium is located at a position where is not covered by the angle of the electrocardiogram, it might be missed. Therefore, it will be helpful to be able to obtain electrocardiograms at different angles so as to comprehensively understand the whole heart and thus accurately judge the heart disease.

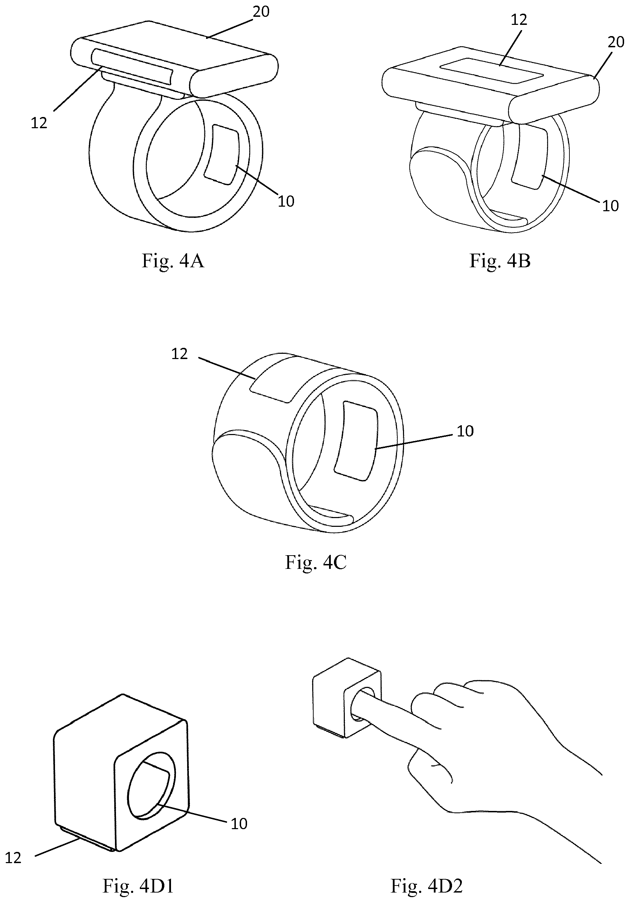

Preferably the position for mounting thee finger-worn structure is to locate on the intermediate phalanx or the proximal phalanx of the finger, so as to avoid the finger-worn structure from falling off the finger tip. For example, the finger-worn structure can be configured to have a ring shape, as shown in Fig. 4A, or to be a flexible belt with a housing, as shown in Fig. 4B, or to be a flexible belt only, as shown in Fig. 4C, or to have a C shape, there is no limitation. No matter in which type, the diameters of the finger-worn structures all can be implemented to be adjustable, so as fit the finger and thus ensure the contact of the electrode with the skin. For example, the ring type structure can be configured to have an adjustment mechanism, the belt can be configured to have multiple fixing points, e.g., via using Velcro, so that the user can adjust the structure by himself/herself. In addition, it also can be implemented to be a clamp to clamp the phalanx or finger tip, and due to the elasticity of the clamp, it still can be fixed well.

Furthermore, the finger-worn structure also can be configured to be a sleeve for mounting at the finger tip (as shown in Fig. 4D1-4D2), namely, an indentation structure for receiving the finger, e.g., it can be implemented to be circular shape or a hole. The first electrode is mounted on the inner surface of the indentation structure. The inner surface of indentation is configured to conform to the shape of finger, so that when the finger is inserted therein, the contact between the first electrode and the skin can be achieved. The indentation structure can be made of flexible material, such as, rubber or silicon, so as to achieve the contact; alternatively, it also can be configured to have plastic housing with the inner portion being made of flexible material; or alternatively, it can be configured to include a force-applying mechanism inside the housing for ensuring the contact between the electrode and the skin. Therefore, there is no limitation. This type of device is advantageous to contact different body positions in an easy way, so as to obtain electrocardiograms at different heart angles. Thus, the finger-worn structure of the present invention can be implemented to be many types without limitation.

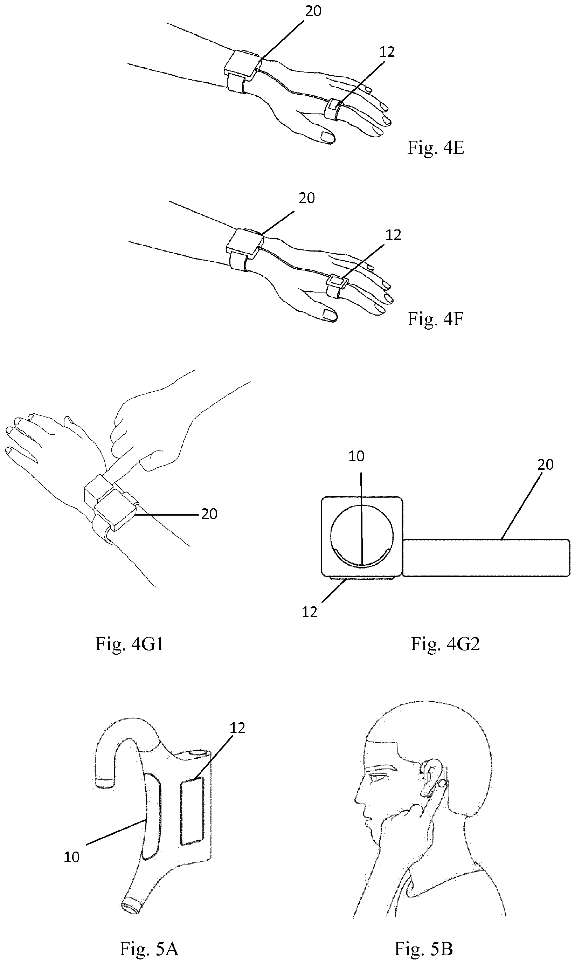

Moreover, in a preferred embodiment, the housing 20 also can be implemented to connect with the finger-worn structure via a connecting wire and carried by a wrist-worn structure, so as to position on the wrist, as shown in Fig. 4E. Accordingly, some hardware, such as, circuitry and battery, which are originally located on the finger-worn structure can be moved to the wrist-worn structure, so as to reduce the burden of the finger. Further, on the surface of the wrist-worn structure and/or a surface of the housing 20 which is not contacted with the wrist, the second electrode can be positioned, so as to provide another contact choice. Alternatively, as shown in Fig. 4F, it also can be implemented that the finger-worn structure and the wrist-worn structure both bear a housing. Therefore, this is no limitation.

In another preferred embodiment, the housing 20 also can be implemented to combine with the finger-worn structure via a pair of connectors, as shown in Fig. 4G1-4G2, electrically and mechanically. In this case, the first electrode 10 contacts the finger of right hand, and the second electrode 12 contacts the skin around the wrist of left hand. And, since the finger-worn structure is positioned on the wrist due to the connection with the housing of the wrist-worn structure, when the user's both hands are positioned on a fixed support, such as, table or desk, the measurement pose will be stable, and thus, the rise of electromyogram can be minimized. Further, through the connection via connectors, the path for sampling electrocardiogram can be shortened, so that the interference from the environment, such as, electromagnetic noises, also can be reduced. Thus, this is also advantageous.

In another aspect of the present invention, the wearable structure is implemented to be an ear-worn structure. Therefore, the wearable ECG measurement device of the present invention is carried by the ear-worn structure and then mounted on an ear of the user. As to the circuitry, it can be accommodated in the ear-worn structure and/or can be contained in a housing combined with the ear-worn structure.

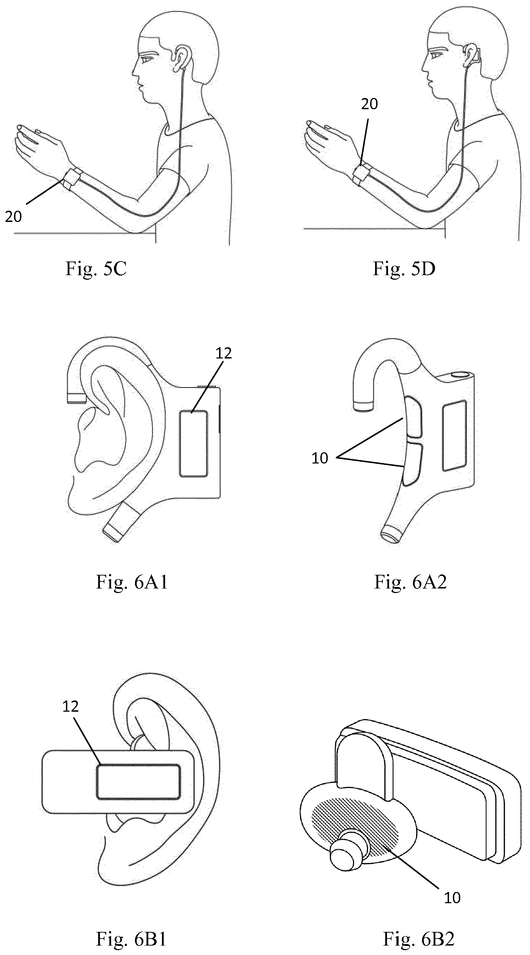

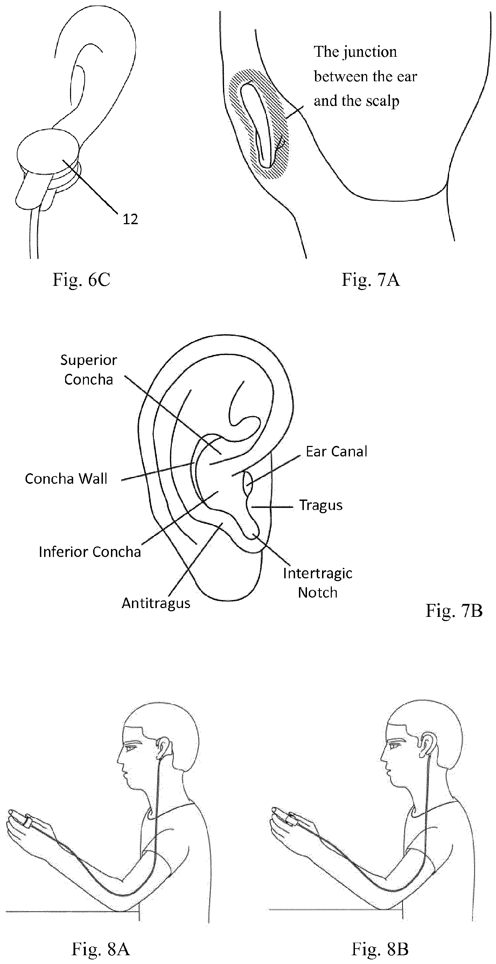

As shown in Fig. 5A, the first electrode 10 is located on a surface of the device where will contact the skin of the ear or around the ear while the ear-worn structure is mounted on the ear. The second electrode 12 is at least located on a surface of the device which will not contact the skin of ear or around the ear while the ear-worn structure is mounted on the ear, e.g., an opposite surface or an adjacent surface, without limitation. Here, additionally, the first electrode 10 can be implemented to be two electrodes, as shown in Fig. 6A2, and one of the electrodes is used as ground or referential electrode, so as to suppress common mode noises, such as, noises from power source. There is no limitation.

Here, the advantage of utilizing ear as the contact position of electrode is that the ear and the area near around the ear are regions with very small electromyogram. And, because positions of the ear and the head are almost unchangeable, even the user moves the body during the measurement, for example, turns the body or rotates the head, the contact between the electrode and the skin still can be remained stable and won't cause too many interferences.

Further, in daily life, compared with other body portion, the ear is a portion which is rarely covered by cloth, and also can be easily and directly contacted as needed. And, the ear and the area near the ear also have less hairs, so that the contact between the electrode and the skin can be easily achieved. Therefore, for user, this is a convenient choice.

In practice, as shown in Fig. 5B, the user only needs to lift up the hand to contact the second electrode, and then, the electrocardiogram can be acquired conveniently.

In a preferred embodiment, the housing 20 also can be connected to the ear-worn structure via a connecting wire, and carried by a wrist-worn structure, so as to be mounted on a wrist, as shown in Fig. 5C. Accordingly, some hardware, such as, circuitry and battery, which are originally located on the ear-worn structure can be moved to the wrist-worn structure, so as to reduce the burden of the ear. Further, on the surface of the wrist-worn structure and/or a surface of the housing 20 which is not contacted with the wrist, the second electrode can be positioned, so as to provide another contact choice. Alternatively, as shown in Fig. 5D, it also can be implemented that the ear-worn structure and the wrist-worn structure both bear a housing. Therefore, this is no limitation.

Here, the ear-worn structure can be varied as needed. For example, it can be implemented as the common ear-worn structures found in daily life, such as an ear-hook, an ear plug, or an ear clamp shown in Fig. 6A1-6C, so that there is no need for the user to re-learn how to use, and just like wearing an earphone, the installation of electrode can be easily completed. And, in this case, the contact between the electrode and the skin will be achieved by the ear-worn structure, namely, the user doesn't need to apply force, so that the rise of electromyogram can be reduced and thus obtain high quality signals.

Particularly, in a preferred embodiment, the ear-worn structure is implemented to utilize magnetic force to attach to the ear. For example, it can be two elements which magnetically attract across the ear, and the electrode can be implemented to locate on one of the elements. Here, the two elements can be both magnetic, or only one is magnetic and the other is attractable by magnetism, without limitation. As to the magnetism, it can be achieved by inserting magnetic material into the element or by making the element directly of magnetic material. And, identically, the material attractable by magnetism also can be inserted therein or directly formed.

As to which part of ear can be used to acquire electrocardiogram, there is no limitation. It can be any position of the ear, such as, the canal, the lobe, the concave side, and the convex side, e.g., the inferior concha, the area near the opening of canal, and/or helix, and also can be the area around the ear, such as, the junction between the ear and the scalp. All these positions can be contacted by the electrode to acquire ECG signals.

Here, there is one position that should be mentioned. Please refer to Fig. 7B, which shows the physiological structure of the auricle (also named as pinna). Within the concave side, around the superior concha and the inferior concha, there is a perpendicular area which is extended from the concha floor (namely, a surface parallel to the scalp) up to the antihelix and antitragus, and called as concha wall. This naturally physiological structure just provides a continuous surface perpendicular to the concha floor. And, the intertragic notch which is adjacent to the antitragus and the tragus also provide a contact area perpendicular to the concha floor.

When the contact of electrode is aimed at this continuous perpendicular area, the direction of force for fixing the electrode will be parallel to the concha floor (namely, perpendicular to the concha wall). Especially, when it is implemented as an ear plug, the against force between the ear plug and the structures at the concave side of auricle will naturally achieve a stable contact between the electrode and this perpendicular area. Therefore, it is very convenient.

Furthermore, the contact at the convex side is also advantageous. Generally, the ear-hook structure includes one part at the concave side and another part at the convex side which have mutual forces toward each other for fixing the structure on the ear. Therefore, if the electrode is selected to contact the convex side, it will just match to the directions of the forces provided by two parts of the ear hook structure, and thus, the contact of electrode can be achieved naturally and stably.

Since the contact position is ear, the ear-worn ECG measurement device of the present invention is also suitable for combining with earphone, such as wired or wireless earphone. In this case, except for blending the ECG measurement more into the daily life, the sounding function of earphone can be given to full play. For example, it can use sound or voice to provide user the analysis results, such as, notify the abnormal of electrocardiogram, or periodically inform user to record the electrocardiogram, so as to facilitate the operation.

When it is implemented to combine with the earphone, not only the single-ear type, but also the dual-ear type can be used. For example, the earphone with microphone is usually implemented as single-ear type, similar to the above-described embodiments, and the earphone for listening music is usually implemented as dual-ear type, and in this case, it only needs to locate the electrode on one side of the ear-worn structure, without limitation.

It should be noted that both ears can be selected to position the electrode. However, according to the experiment results, the contact position of the second electrode does have influence in signal quality. When it is implemented to be the left limb contacts the second electrode or the second electrode is positioned on the left limb, the acquired electrocardiogram will have much better signal quality than with the right limb. Among these, the best signal quality is when the two electrodes respectively contact the left ear and the left limb. Accordingly, when the ECG measurement is implemented by contacting the ear, it is preferable to contact the other electrode with the left limb, so as to avoid from poor signal quality and thus misjudgment.

Particularly, in another aspect of the present invention, further, the first electrode and the second electrode can be respectively implemented to be carried by finger-worn structure and ear-worn structure for achieving the contact with the skin, as shown in Figs. 8A-8B. Accordingly, the user only needs to put the structure on the finger and the ear, the installations of electrodes for ECG measurement can be completed in a very convenient way. And, since the contacts of both electrodes with the skin are achieved by wearable structure, the electromyogram arisen from muscle tension can be reduced to minimum.

Here, as shown in Figs. 8A-8B, it can be implemented to have one housing combined with single wearable structure, or to have housings combined with both wearable structures, without limitation. And, the circuitry also can be accommodated in any of the wearable structures or housings based on the real demands.

Besides, except for utilizing finger-worn structure to cooperate the ear-worn structure, other positions also can be employed to mount the electrode, such as, the neck, the shoulder, the upper arm, the forearm, and the chest. For example, a neck-worn structure, e.g., necklace or collar, can be utilized to position the electrode near the neck, the shoulder and/or the chest; an arm-worn structure or wrist-worn structure can be utilized to position the electrode on the upper limb; and a chest band or a patch can be used to position the electrode on the chest. All these are convenience, too. Therefore, any position where can be used to position the electrode for acquiring electrocardiograms will be belonged to the scope of the present invention, without limitation.

In still another aspect of the present invention, the wearable structure is implemented to be a wrist-worn structure. Accordingly, the device is carried by the wrist-worn structure and located on a wrist of the user, and the circuitry is accommodated in the wrist-worn structure and/or an additional housing combined therewith.

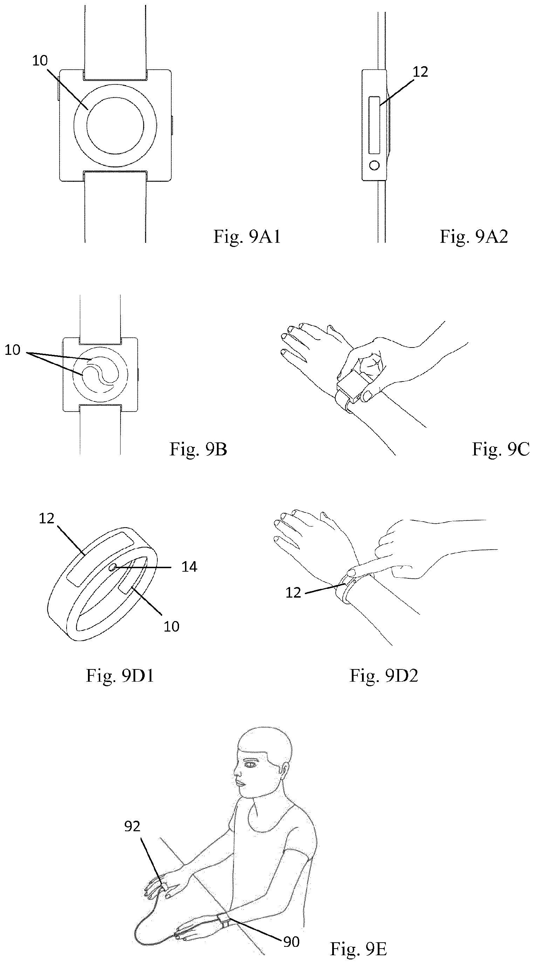

As shown in Figs. 9A1-9A2, the first electrode 10 is located on a surface of the device which will contact the skin near the wrist while being worn thereon. And, the second electrode 12 is at least located on a surface of the device which will not contact the wrist, such as, the surface opposite to or adjacent to the surface for mounting the first electrode, without limitation. Here, the first electrode 10 also can be implemented to be two electrodes, as shown in Fig. 9B, and one of the electrodes is used as ground or referential electrode, so as to suppress common mode noises, such as, noises from power source. There is no limitation.

The reason for selecting the wrist to mount the ECG measurement device is that, for a general user, wearing the wrist-worn structure is just like wearing a watch which is familiar without re-learning. Therefore, after the user wears the wrist-worn device on the wrist, the installation of the first electrode is completed. Then, when there is the need to record the electrocardiogram, the user only needs to contact the second electrode with a skin portion other than the limb wearing the device, and the acquisition of electrocardiogram can be achieved. The whole process is simple, easy, natural and convenient. Besides, since the contact between the first electrode and the skin is achieved by the wrist-worn structure, the muscle tension arisen from applying force can be reduced, and thus, the influence thereof on the ECG signals also can be reduced.

In practice, as shown in Fig. 9C, the user only needs to contact the second electrode by the other hand, and the sampling loop for acquiring electrocardiogram can be achieved conveniently. Except for combining an additional housing, it also can be implemented to contain the circuitry in the wrist-worn structure, as shown in Figs. 9D1-9D2. There is no limitation.

Particularly, in another aspect of the present invention, as shown in Figs. 9E-9F2, the first electrode 10 and the second electrode 12 can be respectively implemented to be carried by wrist-worn structure 90 and finger-worn structure 92 for achieving the contact with the skin. Accordingly, the user only needs to put the structure on the wrist and the finger, the installations of electrodes for ECG measurement are completed in a very convenient way. And, since the contacts of both electrodes with the skin are achieved by wearable structure and if the user can place the hands on a stable surface, such as, table or desk, the electromyograms arisen from muscle tension will be able to be reduced to minimum.

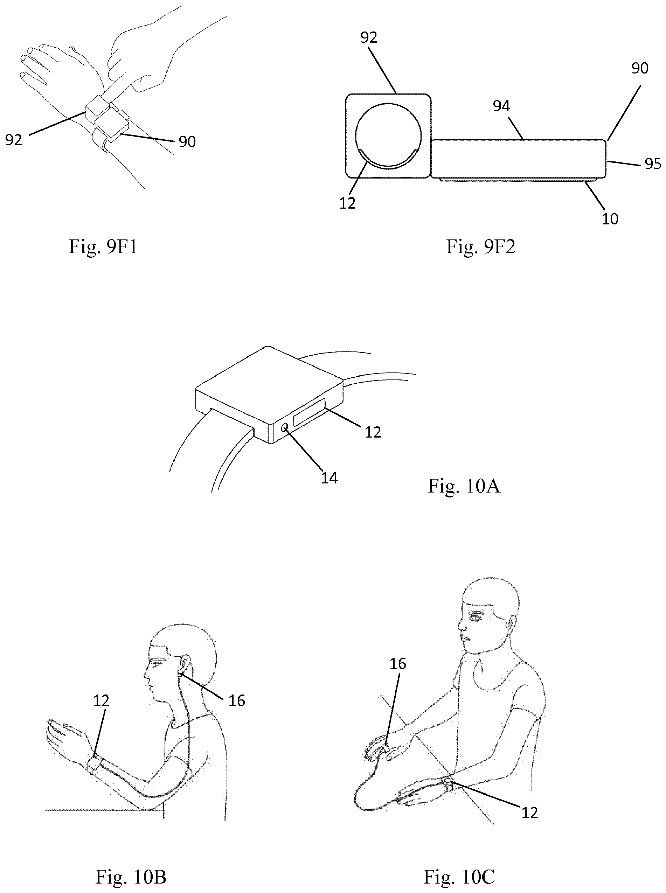

Besides, it also can additionally mount a third electrode on a surface of the wrist structure which does not contact the skin of wrist, e.g., the surface 94 or surface 95 in Fig. 9F2, so that even the user doesn't wear the finger-worn structure, it still can utilize the first electrode 10 and the third electrode to acquire electrocardiogram, so as to provide another operation selection.

Furthermore, the wrist-worn ECG measurement device of the present invention also can include a connection port 14, as shown in Figs. 10A-10C, for connecting the third electrode 16 via a connecting wire. Here, in a preferred embodiment, the third electrode is implemented to replace the second electrode, for example, it can be that the second electrode will be disabled automatically as the third electrode is connected to the connection port; or alternatively, a switch can be utilized, so that the user can decide which electrode to be enabled, without limitation. Also, the third electrode 16 also can be implemented to be carried by a wearable structure, such as, an ear-worn structure (Fig. 10B), a finger-worn structure (Fig. 10C), a glasses structure, or a wrist-worn structure.

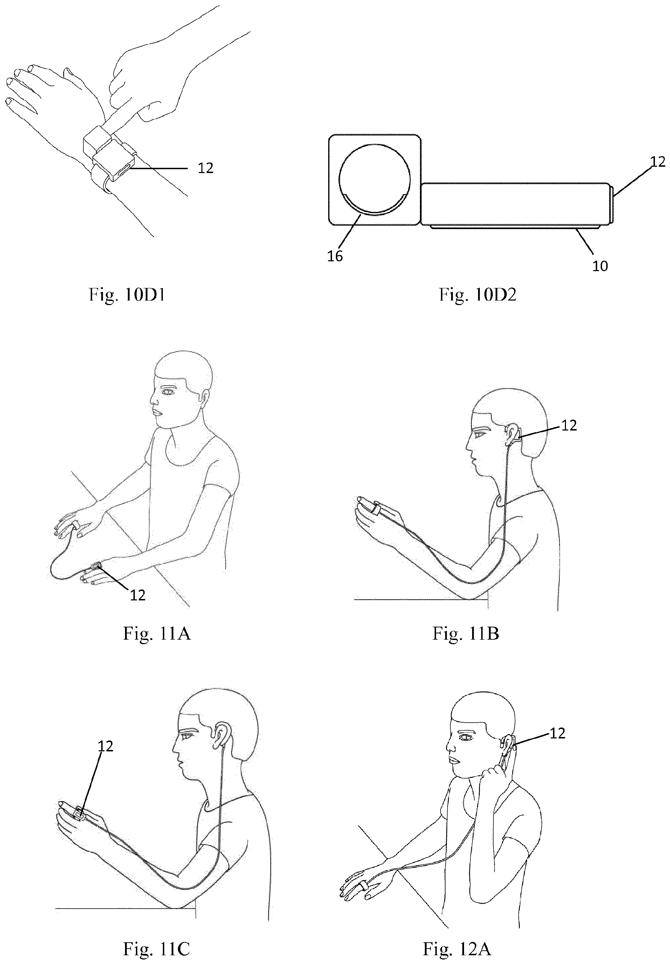

Alternatively, the third electrode 16 also can be implemented to connect through a connector, as shown in Figs. 10D1-D2. In this case, the first electrode 10 contacts the wrist as the user wears the wrist-worn structure, and the third electrode 16 is located inside the finger-worn structure. Since the finger-worn structure and the wrist-worn structure are combined electrically and mechanically together through a pair of connectors and the combination of two structures will be located on the wrist as an integral object, the pose during measurement is very stable, so as to facilitate the acquisition of high quality signals.

When the third electrode is connected via a connecting wire, as shown in Figs. 10B-10C, compared with the second electrode, the device of the present invention can provide more choices for contact, so as to obtain electrocardiograms at different angles, For example, when using the second electrode, the electrocardiogram is obtained via two hands (the wrist wears the wrist-worn structure and the other hand contacts the second electrode), and when using the third electrode, it can utilize an ear-worn structure to contact the ear, so as to obtain electrocardiogram at different angle (e.g., the left hand wears the wrist-worn structure and the left ear wears the ear-worn structure).

As mentioned above, a lesion might be located at any portion of the heart. For example, the examination of myocardial infarction has to check the ST shift in the electrocardiogram which is caused from the damaged myocardium. However, if the damaged myocardium is located at a position where is not covered by the angle of the electrocardiogram, it might be missed. Electrocardiograms at different angles are necessary.

Accordingly, other than using the second electrode on the surface of the device, by using the extended third electrode, the present invention provides an additional way to acquire electrocardiogram, such that the user can simply connect the third electrode to the device and more heart information can be obtained.

Moreover, the extended third electrode also provides other advantages in usage.

In the present invention, the arrangement of the second electrode makes the user can simply and rapidly acquire electrocardiogram by touching the electrode on the surface. As to the third electrode, it provides another choice to obtain electrocardiogram in a more stable way. Since the third electrode is carried by a wearable structure to contact the skin, the factors which influence signal quality the most, such as muscle tension and hand shaking, all can be eliminated, so as to facilitate the acquisition of stable and high quality ECG signals.

Further, compared with the second electrode which should be contacted by hand, the third electrode, which is extended via the connecting wire as shown in Figs. 10B-10C, makes the user can select a position with stronger ECG signals, such as, a position closer to heart, so as to reduce the influence of noises. For example, the EMG signals with the same amplitude could be eliminated within an ECG signal having larger amplitude (stronger ECG signal), but might not be identified within an ECG signal having smaller amplitude (weaker ECG signal) so as to cause misjudgment. Thus, through the third electrode, the accuracy of the analysis results can be improved.

Therefore, the wrist-worn ECG measurement device of the present invention provides two operation modes, a first operation mode and a second operation mode. In the first operation mode, the first electrode and the second electrode form a first ECG signal acquisition loop for acquiring a first kind of electrocardiograms, and in the second operation mode, the first electrode and the third electrode form a second ECG signal acquisition loop for acquiring a second kind of electrocardiograms. Through the selectable operation modes, it can be ensured that the acquired electrocardiograms to have high and stable quality without being influenced by different environments and habits.

Similarly, the finger-worn ECG measurement device and the ear-worn ECG measurement device of the present invention also can be configured to have a connection port for connecting an extended third electrode.

For example, as shown in Fig. 11A, the finger-worn ECG measurement device is configured to connect a finger-worn third electrode, and as shown in Fig. 11B, the ear-worn ECG measurement device is also configured to connect a finger-worn third electrode. In these two cases, the original hand-touching operation is replaced by the finger-worn structure which can actively force electrode to contact the skin, so that the unstable factors which might be arisen by hand touching operation can be eliminated, for facilitating of obtaining more stable signals. Further, as shown in Fig. 11C, the finger-worn ECG device also can be connected with an ear-worn third electrode, so that, except for providing a measurement method without applying force by user, this also provides a different angle of electrocardiogram from two hands operation. Therefore, there is no limitation.

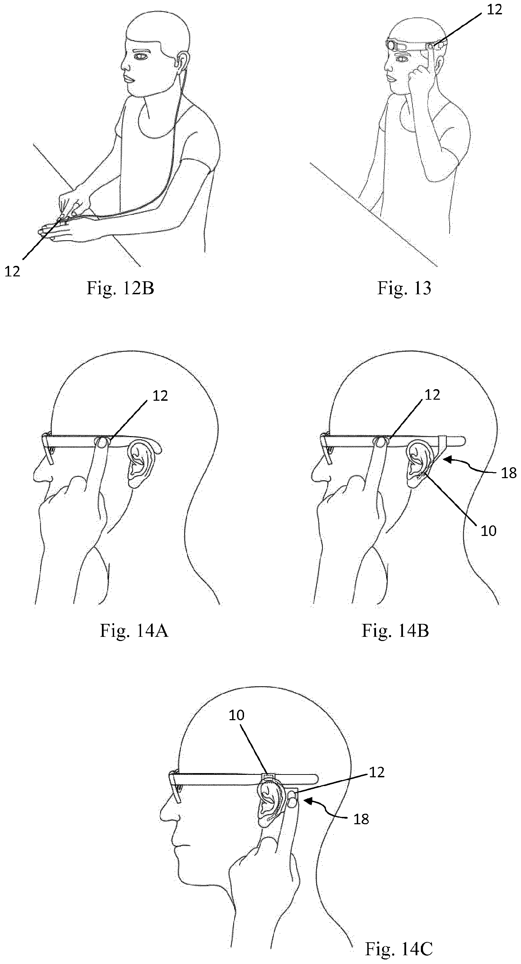

Furthermore, the wearable ECG measurement device of the present invention also can be implemented to acquire the first kind of electrocardiograms and the second kind of electrocardiograms at the same time, as shown in Fig. 12A and Fig. 12B. Namely, during the measurement, the first electrode will respectively form a loop with the second electrode and the third electrode to acquire ECG signals. Therefore, the user can select different operation modes depending on different demands so as to obtain more appropriate heart information.

Moreover, in another aspect of the present invention, the wearable structure is implemented as a head-worn structure. Accordingly, the ECG measurement device is carried by the head-worn structure and mounted on the head of the user, and the circuitry is accommodated in the head-worn structure and/or an additional housing combined therewith.

As shown in Fig. 13, the first electrode is located on a surface of the device which will contact the skin of the head while being worn thereon. And, the second electrode 12 is at least located on a surface of the device which will not contact the head, for contacting with an upper limb (e.g., finger, and arm), neck or shoulder, so as to achieve an ECG signal acquisition loop. It should be noted that the position of the second electrode can be selected to be different. For example, the second electrode can be located at a surface opposite to or adjacent to the surface of the first electrode for being touched by an upper limb, or can be located on a finger-worn structure, a wrist-worn structure or an arm-worn structure to contact the skin of the upper limb; or alternatively, the second electrode also can be located on an outer surface of an ear-worn structure which is connected with the head-worn structure for being touched by the upper limb; or alternatively, the second electrode also can be extended to contact the neck and/or shoulder through, for example, a neck-worn structure, e.g., necklace or collar, or extended to the chest through, for example, a chest band or patch. All these can be used to acquire ECG signals, and there is no limitation.

Here, the head-worn structure also can be implemented to different forms, for example, a band, a headgear, a frame with adjusting mechanism, or a pair of glasses. There is no limitation and the point is to achieve the contact of the electrode with the skin of the head.

Among these, the glasses structure is a very convenient choice. Generally, when the glasses structure is worn on the head, it will contact many positions, for example, but not limited, the nose pad will contact the nasal bridge, the root of nose, and/or the area between two eyes, the front portions of the glasses temples will contact the areas around the temples, the rear portions of the glasses temples will contact V shaped valleys between the head and the ears, and the end portions of the glasses temples which are located behind the ears will contact the concave sides of the auricles. Therefore, when the first electrode is mounted on a position of the glasses structure which will contact the skin, the contact between the first electrode and the skin will be completed as the glasses is worn on the head. Then, it only needs to mount the second electrode 12 on a position of the glasses structure which can be contacted by an upper limb, as shown in Fig. 14A, the acquisition of ECG signal can be achieved. Accordingly, not only the measurement can be performed in a very convenient way, the ECG measurement also can be bent into the daily life, so as to increase the usage intention.

Moreover, for example, if there is the need to monitor electrocardiogram for long period of time, the second electrode can be implemented to be carried by a wearable structure, such as, finger-worn structure, wrist-worn structure, arm-worn structure, neck-worn structure, shoulder-worn structure, chest band, or patch, for being fixed on another body portion. It can be changed in accordance with the needs without limitation.

It should be noted that the glasses structure refers to a wearable structure which is supported by the auricles and the nose and will contact the skin of the head and/or the ear. Therefore, it is not limited to the general glasses and also can include its transformation, for example, it can be a structure which provides opposite forces at the two sides of the head; or it also can have an asymmetric structure, such as, one temple is implemented to have bent portion at the end and the other temple is implemented to be straight; or it also can be a pair of glasses without lens. There are many kinds of possibilities without limitation.

Moreover, the material of the glasses structure also can have different choices. Other than the general hard material, it is also preferable to employ flexible material so as to increase the stability of electrode contact and further make the user feel more comfortable. For example, it can employ memory metal or flexible plastic to form the frame; and/or it also can be implemented to mount elastic rubber or silicon at the position of electrode, so as to stabilize the contact thereof.

The combination manner of the electrode with the glasses structure also can have many possibilities. For example, the electrodes and the circuitry (e.g., processor, battery, wireless module etc.) can be directly embedded in the glasses structure, such as, temples and/or rims of lens. Alternatively, an additional structure can be used to achieve the arrangement of electrode and the circuitry, for example, as shown in Fig. 14B, the additional structure 18 is extended from one side temple of the glasses, so that the first electrode 10 can contact the lower portion of the concave side of auricle while the second electrode 12 can be mounted at the outer surface of the glasses structure. Alternatively, as shown in Fig. 14C, the additional structure 18 is extended from one of the glasses temples, so that the first electrode 10 can contact the area around V shaped valley (here, the first electrode can be implemented to contact the scalp, the V shaped valley, and/or the concave side of auricle without limitation) while the second electrode 12 can be mounted on the outer surface of the additional structure for being touched. Consequently, there are many kinds of possibilities, and the circuitry can be set to partially or fully contain in the glasses structure and/or the additional structure without limitation. Besides, the additional structure can be implemented to be removable, so that the user can select to attach the additional structure to the glasses structure as needed. There is no limitation.

When the head-worn structure is implemented to be the glasses structure, preferably, it is employed to provide the functions of earphone and/or microphone, for example, through mounting an acoustic component and/or a sound receiving element (e.g., microphone) thereon, or to have the earphone extended thereout. Particularly, the adopted acoustic component or earphone can be not only air conduction type but also bone conduction type, for example, a bone conduction speaker can be directly mounted at a position of the glasses structure which will contact the head bone, or it also can employ a bone conduction earphone extended from the glasses structure. There is no limitation.

In addition, the device of the present invention also can be configured to communicate with a portable electronic device, for example, to execute wired or wireless communication via, e.g., earphone socket or Bluetooth, with a portable electronic device, such as smart phone or tablet. Thus, when there includes the acoustic component (air conduction or bone conduction type) and the sound receiving element, the device of the present invention can be used as a hand-free handset. Further, through setting vibration module, acoustic component (air conduction or bone conduction type), display element, and/or light emitting element etc, the device of the present invention can be used as an information providing interface of the portable electronic device, such as, to notify the call or message. Accordingly, the device can be bent in the daily life even more. As to how the notification presents to the user, it can be in different manners, such as, sound, vibration, light, display on the lens, without limitation.

Similar to ears, head also has the characteristic of being not easy to rise EMG signals which will influence ECG signals, so that the head is also suitable to position the ECG electrode. Further, since the head-worn structure is employed, it will be preferable to also mount EEG electrodes thereon so as to acquire EEG signals. For example, it only needs to mount at least two electrodes at the inner side of the head-worn structure/glasses, or to respectively mount an EEG electrode on the head-worn structure and another EEG electrode on an ear-worn structure extended from the head-worn structure, for contacting the EEG sampling points on the scalp, such as, the common used Fp1, Fp2, O1, O2, A1, A2, or any point according to the international 10-20 system. Accordingly, without increasing any burden, the device can provide more measurement functions with advantages.

Furthermore, it also can be implemented that the ECG electrode which contacts the skin of head is shared to be used as EEG electrode, so that the first electrode can cooperate with the second electrode to form ECG signal acquisition loop and with another EEG electrode to form EEG signal acquisition loop.

Alternatively, the share of electrode can be implemented in a different manner. For example, both electrodes are used to acquire ECG signals and EEG signals, namely, ECG signals and EEG signals are acquired by one identical signal acquisition loop. The reason why this is workable is that the amplitude of ECG signals is ranged in millivolt level, and that of EEG signals is ranged in microvolt level, so that even employing the identical signal acquisition loop, they still can be separated.

In practice, for example, it can employ one electrode to contact the head while another electrode is used to simultaneously contact the head and the hand. Here, take the most common used metal electrode plate as example, the electrode for simultaneously contacting the head and the hand can be implemented as two electrically connected electrodes for respectively contacting the head and the hand, or also can be implemented as two portions of one single electrode for respectively contacting the head and the hand, such as, the inner side of the electrode mounted on the head-worn structure contacts the head and the outer side contacts the hand. Therefore, there is no limitation.

Accordingly, EEG electrodes are also suitable to mount on the ear-worn structure of the present invention, as shown in Figs. 5-6 and Fig. 8. First, on the ear and the scalp near the ear, it is where can detect the activity of cortex, e.g., temporal lobe. Then, in the field of EEG measurement, ears are usually regarded as suitable positions to place referential electrode due to the discontinuous physiological structure and position from the head, which makes ears not easy to be influenced by brain activity. Thus, it is common to combine the referential electrode with the ear-worn structure. Accordingly, the ear-worn ECG measurement device of the present invention is also suitable to combine with EEG electrodes for acquiring EEG signals, and identically, the electrode also can be implemented to shared electrode, namely, to simultaneously take the first electrode as EEG electrode.

Moreover, in the embodiments of Figs. 5-6 and Fig. 8, except that the two EEG electrodes can be both mounted on the ear-worn structure, it also can additionally employ a head-worn structure for mounting the EEG electrode. Accordingly, EEG signals can be acquired by the EEG electrodes respectively on the ear-worn structure and the head-worn structure, and ECG signals can be acquired by one ECG electrode mounted at the inner side of the ear-worn structure and another ECG electrode mounted at the outer side of the ear-worn structure (Figs. 5-6) or on the finger-worn structure (Figs. 8). There are many possibilities.

The glasses structure also can be used to achieve the acquisitions of EEG signals and ECG signals. As mentioned above, when the glasses structure is worn on the head, it will contact the head and/or the ear at many positions, such as, nasal bridge, root of nose, the area between two eyes, temple, the V shaped valley between the head and the auricle, and the concave side of the auricle, or if the glasses temple is extended to the rear of head, it will contact the area around the occipital bone. Thus, it only needs to mount two EEG electrodes and the first electrode (or one of EEG electrodes is used as ECG electrode at the same time) on the glasses structure at positions which will contact the skin, and then, by cooperating with the second electrode located on the outer surface of the glasses structure or extended from the glasses structure, not only ECG signals, but also EEG signals can be acquired, which is very convenient.

Particularly, the glasses structure can further combine with an ear-worn structure for mounting the first electrode and the second electrode, for example, an ear plug or ear clamp can be extended from the glasses structure, or the glasses structure can be configured to have a connection port for electrically connecting the ear plug or ear clamp, or the ear-worn structure also can be sleeved on the glasses temple. Accordingly, there will have more possibilities. For example, the first electrode can be mounted on the glasses structure, and when there is the need of measurement, the user can then connect the ear-worn structure to the connection port and acquire ECG signals via the second electrode at the outer surface of the ear-worn structure. Alternatively, the first electrode can be mounted inside the ear-worn structure to contact the ear, and the second electrode is located on the outer surface of the glasses structure. Alternatively, the first electrode and the second electrode both can be mounted on the ear-worn structure which is combined with the glasses structure for being mounted around the ear, and this architecture provides the possibility to use a common glasses structure that everyone to additional mount the physiological detection element (in this case, the ear-worn structure with two ECG electrodes), which provides a convenient way. Therefore, there is no limitation

In practice, because the ECG measurement device of the present invention is a wearable device, it is also possible to perform continuous ECG acquisition in a convenient way.

First, based on the wearable structure, the device can be worn on user's body without burden, and thus, it will be suitable to use in the daily life. For example, the user can wear the device on the ear, the finger or the wrist in daily life, and when there is the need, such as, the user feels the heart problem, he/she can start the ECG measurement promptly. Alternatively, the user also can perform the ECG measurement periodically every day, so as to understand the heart condition effectively.

Especially, arrhythmia always occurs without warning, so that through this kind of wearable ECG measurement device, it is helpful to record the electrocardiogram during arrhythmia in real time or the electrocardiogram when the users feels hart problem, thereby facilitating the doctor to diagnose the syndrome.

For example, no matter the device is implemented to be finger-worn, ear-worn, head-worn, or wrist-worn type, or glasses type, the user can acquire and record electrocardiogram in real time by touching the second electrode with the hand while feeling heart problem or simply wanting to record the heart condition, as shown in Fig. 2A, Fig. 5B, Fig. 9C and Fig. 14. Alternatively, when the second electrode is also carried by a wearable structure or it is a situation to employ the third electrode, two electrodes are already contacted with skin, so that the user only needs to start the measurement, such as, by pressing the start button, and the electrocardiogram can be recorded in real time. No matter in which situation, the operation is very simple and convenient.

Here, the device of the present invention also can be set to automatically record a preset period of time, e.g., 30 seconds or 1 minute, after being activated, so as to provide the user an easier way to record the electrocardiogram during heart problem, such as, arrhythmia, in real time.

Furthermore, the user also can be selected to perform long-term recording of continuous electrocardiograms, especially when the two electrodes are already worn on the body via the wearable structures. And, through analyzing the continuous electrocardiograms, there are more information can be obtained, which is also advantageous.

For example, based on the continuous electrocardiogram, information about continuous heart beat sequence can be obtained, and thus, could be used to perform HRV (Heart Rate Variability) analysis. HRV analysis is the main method to observe ANS (Autonomic Nervous System) activity. Through the analysis results, it can understand ANS activity in detail, for example, the activity of sympathetic nervous system (SNS), the activity of parasympathetic nervous system (PSNS), the balance of ANS, and the activity level of ANS. More and more studies show that many diseases, such as, headache, gastrointestinal discomfort, high blood pressure, insomnia, depression, might be caused by autonomic disorders. Through long term HRV analysis, it can reveal the variations of ANS activity during daily life, and thus, can understand that if any particular kind of behavior or emotion causes ANS disorder and if ANS disorder is the cause of above-mentioned diseases.

And, because the device of the present invention is wearable type, through the information providing unit, the HRV analysis results can be provided to the user in real time, and thus, the user can recognize that what kind of behavior or emotion causes the imbalance of ANS. Furthermore, through this design, the user can also accordingly adjust the physical and mental conditions in real time, e.g., to relax physically and mentally, thereby knowing that if the ANS becomes more balance.

Besides, when using during sleep, through performing HRV analysis of continuous electrocardiograms, it also can understand the physiological conditions during sleep, such as, sleep cycle, sleep quality etc., which is convenient.

It should be noted that after acquiring ECG signals, the device of the present invention also can be implemented to store ECG signals first, and then process the stored data after the measurement ends, for example, output to a computer for storage and analysis. Alternatively, since the present invention includes the information providing unit, the related information and/or analysis results, e.g., average heart rate, HRV analysis results, also can be provided to the user via the information providing unit. Alternatively, the information providing unit also can be configured to output the stored ECG signals and/or data to an external device, e.g., mobile phone, tablet, so that the external device can perform a real time display and/or analysis.

Moreover, the device of the present invention also can be utilized for the user to perform breathing training. Through the wearable type, the device of the present invention can acquire continuous electrocardiogram and thus obtain the time sequence of heart beat intervals, namely, heart beat sequence. And, through analyzing the heart beat sequence, it will be able to obtain information about respiratory sinus arrhythmia (RSA). RSA is a naturally occurring variation in heart rate that occurs during the breathing cycle since heart rate is controlled by ANS and breathe can influence ANS. Generally, during inspiration, heart rate will increase, and during expiration, heart rate will decrease. Therefore, through observing RSA, it will be able to reveal the breathing pattern and ANS activity.

As known, breathing not only is controlled by ANS, but at the same time, also can be changed consciously in a particular range. Accordingly, it will be able to achieve a relaxation effect in physical through consciously adjusting the breathing to influence ANS. Wherein, the decreased breathing rate will suppress SNS activity, and the increased breathing rate will oppositely encourage SNS activity. For example, the general breathing rate of adult is ranged between 10-18 times per minute, and when the breathing rate is lowered to 5-8 times per minute, it will be helpful to increase PSNS activity. And, when the ratio of expiration/inspiration period increases, namely, the duration of expiration is longer than that of inspiration, the PSNS activity also can be increased.

Generally, the breathing training is performed by providing user a breathing guiding, which includes a breathing pattern that can help to relax the physiological condition. For example, the breathing guiding might have a guiding rate of 5-8 times per minute, and/or of longer expiration duration within a normal breathing pattern, so as to guide the user to lower down the breathing rate and/or increase the expiration duration, thereby suppress SNS activity and thus increase PSNS activity, which result in releasing from stress and relaxing.

ANS disorder is also one of the causes of arrhythmia. Since one of the purposes of using the present device is to record the electrocardiograms during arrhythmia, the breathing guiding function which can guide the user to change the balance of ANS through controlling respiration, will be able to help user to relief the syndrome. It becomes more meaningful by the complement therebetween.

When using the wearable ECG measurement device of the present invention to perform breathing training, the user only needs to wear the device and keep two electrodes contacting the skin. During the breathing training, the information providing unit will provide the breathing guiding signal to the user, and the user follows the guiding to adjust the breathing pattern thereof. Further, the information providing unit also can provide the physiological variations during breathing guiding, for example, the variations of SNS activity and PSNS activity, the variations of heart rate, and the variations of user's real breathing pattern, for being the reference for the user to performing the breathing training.

Here, because performing breathing training will take longer time, it is preferable to select electrodes which can be worn on user's body without hand operation. For example, it can utilize the second electrode carried by the wearable structure, or utilize the third electrode to cooperate with the first electrode (which is also carried by wearable structure) to acquire

ECG signals, and thus, the user can perform the breathing training in an easier way.

Besides, the breathing guiding signal also can be implemented to be a dynamically adjustable guiding signal, which will adjusts in accordance with the breathing pattern derived from the heart beat sequence. Namely, through the real time breathing pattern of the user, it can understand that how is the breathing rate and/or if the breathing rate locates in a suitable range facilitating relaxation, and based on these, the guiding signal can be adjusted dynamically. Therefore, the user can achieve the effect of breathing training in a more comfortable way.

Alternatively, it is known that enlarging the amplitude of RSA is helpful to trigger relaxation response, relief the accumulated stress and thus increase the ratio of PSNS activity/SNS activity. Therefore, through observing the variation pattern of the user's heart rate, it can be implemented to guide the user to expiration at the moment that heart rate starts to rise, and to guide the user to inspiration at the moment that heart rate starts to drop, thereby enlarging the amplitude of RSA and thus achieving the purpose of relaxation.

Moreover, through performing a frequency domain analysis to the heart beat sequence, it also can reveal the coherence and synchronization between the respiration and heart rate. Better coherence and synchronization between respiration and heart rate represent that the heart rhythm is organized and coordinated better, which, in most conditions, represents that the body is in a more relaxed and stable state. Therefore, if these information can be provided to the user during the breathing training, it will be helpful for the user to change the physical condition by consciousness.

When the device also includes EEG electrodes to acquire EEG signals, it will be able to know the user's physical condition by observing the synchronization among heart rate, respiration and electroencephalograms. According to studies, inspiration and expiration will cause the fluctuations of blood in the vessels, and the fluctuations will arrive brain with the blood flow, so as to cause the fluctuations of brainwaves in low frequency section, e.g., lower than 0.5 Hz. Accordingly, it also can reveal the breathing pattern via observing brainwaves. Furthermore, the sinus node of heart and the vascular system are also controlled by ANS, and ANS also will feedback the changes of heart rate and blood pressure to the brain via baroreceptor system and thus influence the function and operation of brain, for example, to influence cortex, which can be detected via electroencephalograms. Therefore, these three physiological factors do influence each other, and a great synchronization thereamong represents the human body is under a more relaxed state. This information is also useful for breathing training.

For example, the information providing unit can be configured to provide real time heart rate related information and/or the information about synchronization between respiration and heart rate while providing the breathing guiding signal. Thus, the user can have a real time understanding about the influence of the respiratory adjustment on ANS, for example, if PSNS activity has increased, or if SNS activity has lowered down. Accordingly, the biofeedback process utilizing the breathing training signal can be more efficient.

Furthermore, the HRV analysis results also can be used to represent the effectiveness of breathing training. For example, it can be implemented to perform HRV analysis before and after the breathing training, thereby revealing the influence of breathing training on the ANS. Even more, it also can be implemented to perform a real-time HRV analysis, and thus, provide the real-time ANS activity to the user through the information providing unit, so as to achieve a feedback process, and through this feedback loop, the user can understand his/her physical condition in real time, which facilitates the relaxation.

Because HRV analysis is based on the analysis for a time period, the real-time HRV analysis can be achieved by the concept of moving window. The procedure will be deciding a calculation time period, e.g., 1 or 2 minutes, to be a time window, gradually moving the time window by time, e.g., to calculate every 5 seconds, and then continuously obtaining the HRV analysis results. Besides, it can also be implemented to utilize the weighting concept in calculation, for appropriately increasing the proportion of signals that closer to the analysis time, thereby making the analysis result more close to the real-time physical condition.

The method of the information providing unit providing the breathing guiding signal can be different. For example, it can be implemented to use visual, acoustic and/or tactile type guiding signal without limitation. The visual guiding signal can includes, but not limited, figures, texts, brightness, and/or color, for example, a figure which represents the breathing pattern can be used to guide the user to inhale and exhale. Alternatively, the lighted number of LEDs can be used to represent the inspiration and expiration, too.

Moreover, the acoustic type guiding signal can includes, but not limited, sound variation and voice. For example, the strength of sound can be used to represent inspiration and expiration; or different kinds of sounds also can be used to guide the user to inhale and exhale, such as, birdcalls, the sound of waves, and different music tracks. Alternatively, it can be implemented to tell the user to inhale and exhale, for example, when the breathing training starts, it can pronouns "inhale" and "exhale" in accordance with the breathing pattern of the guiding signal to guide the user, and when the user's breathing pattern has conformed to the guiding signal, it can pronouns "Please remain the breathing rate" and stop the guiding. Thus, there are many possibilities, and can be changed depending on the real situation.

When the device of the present invention is combined with an earphone, the above-mentioned acoustic type guiding will become even more advantageous. And, because the sound and/or voice will directly transmit into the user's ear, it won't influence others people, and due to the concealment, the breathing training can thus be performed conveniently at any location, for example, can be performed during transportation.

Furthermore, when employing tactile type guiding signal, it is preferred to combine with an element that contacts the body, such as, the wearable structure, for proving vibration, and the vibration methods also can have different choices. For example, it can implemented to utilize vibration to notify the user the start points of inspiration and expiration; or it also can be that the vibration generates only when the user's breathing pattern deviates from the target guiding signal too much.

Here, it is advantageous that when employing acoustic and/or tactile guiding signal, the user closes his/her eyes during breathing training which is helpful for relaxation and adjustment of breathing.

In a preferred embodiment, the breathing guiding signal also can be implemented to be outputted to the external device, such as, a smart phone, a tablet, a smart watch, through the information providing unit and the wired/wireless transmission module, and then through the external device, the breathing guiding signal is provided to the user, for performing the breathing training.

Particularly, in another preferred embodiment, the breathing guiding signal is implemented to be generated by the external device and provided to the user. In this case, the external device will receive, from the information providing unit, the information of user's ANS activity or breathing pattern, and then the received information is provided to the user while providing the breathing guiding signal or is used as the basis to adjust the breathing guiding signal. Besides, the external device also can be implemented to store the received information, so as to be the reference for further reviewing.

In addition, the ECG measurement device of the present invention, expect from acquiring ECG signals and EEG signals, also can be configured to include other physiological sensor for being worn on the body and acquiring physiological information.

For example, it can be configured to have at least an optical sensor. Here, the optical sensor means the sensor having light emitting element and light receiving element and acquiring optical signals based PPG (photoplethysmography) principle, such as, via transmission or reflectance manner. The optical sensor also can be mounted on user's body via the wearable structure. For example, the optical sensor can be located on the surface for positioning the first electrode, for being mounted on the user's body, e.g., finger, ear and around ear, wrist, or head etc, together with the first electrode. Alternatively, the optical sensor also can be carried by another wearable structure, for example, it can be carried by an ear-worn structure, which is combined with the glasses structure, for acquiring physiological signals from the ear and/or near the ear. Thus, there is no limitation.

The optical sensor is used to detect the pulses generated by heart beats, and through the continuous pulse variations, it will be able to obtain the heart beat sequence for further analysis. Because only one single optical sensor is enough for acquiring physiological signals, it will be easy and simple to setup by just wearing the wearable structure on, so that it is beneficial for continuous signal acquisition in long-term monitoring.

When the device of the present invention simultaneously have electrodes to acquire ECG signals and optical sensor to acquire heart beat sequence, it will be particularly beneficial for early warning and judgment of arrhythmia. Although the complete arrhythmia information, such as, different types of arrhythmias, e.g., PAC (Premature atrial contractions) occurred at atrium, and PVC (Premature ventricular contractions) occurred at ventricle, should be determined by reviewing the electrocardiograms, it is still capable of revealing arrhythmia characteristics through observing the variations of heart rate, such as, premature beats, AF (Atrial Fibrillation), tachycardia, bradycardia, and pause. Through the architecture of the present invention, the optical sensor can be employed for long-term usage to acquire continuous heart beat sequence, which is used to pre-screen the occurrences of arrhythmia characteristic, and when the arrhythmia characteristic is detected, the user will be informed and notified to perform ECG measurement, so as to further ensure the correctness of detected arrhythmia characteristic, and also obtain more detailed heart-related information.

In practice, when the user wears the wearable structure on the body, such as, finger, ear, wrist, or head, the optical sensor is ready for acquiring continuous pulses and thus heart beat sequence. Then, the acquired heart beat sequence will continuously be compared with preset arrhythmia characteristics, and once a conformation occurs, an arrhythmia possible event will be determined and a notification for prompting the user to perform ECG measurement will be generated. After receiving the notification, the user just needs to simply contact the second electrode to complete the loop for acquiring ECG signals, so that the electrocardiograms of possible arrhythmia event can be acquired immediately. Here, the electrocardiogram can be directly analyzed to know if arrhythmia occurs and to notify the user the result. Alternatively, the electrocardiogram can be transmitted to an external device in real time, such as, smart phone or tablet, for storage and/or analysis, or for storage first and analysis later, for example, to download to a personal computer for analysis. There is no limitation.

Further, the heart beat sequence acquired by the optical sensor also can be used for performing HRV analysis and breathing straining as mentioned above. Since the procedure is similar and the difference is only that the physiological signals is acquired by optical sensor, the descriptions are omitted. In addition, it also can be cooperated with EEG signals to continuously perform the synchronization analysis among respiration, heart rate and EEG signals, so as to provide more information.

Moreover, when the ECG signals and pulses are acquired at the same time, it will be able to obtain the time the pulse wave takes from heart to the position of optical sensor, namely the so-called PTT (Pulse Transit Time). Because PTT is related to the vessel wall stiffness, which influences the blood pressure, it will be able to calculate the reference blood pressure through the particular relationship between PTT and blood pressure.

When ECG signals are acquired by the hand touching the electrode on the outer surface of the device, since the user needs to lift the hand to touch the electrode, no matter the optical sensor acquires signals from which location, e.g., the auricle, near the ear, nasal bridge, the root of nose, the area between two eyes, or the hand for touching the electrode, the relative height of optical signal acquisition location with the position of heart can remain the same. According to hemodynamics, PTT will be influenced by the height difference between the measurement position and the heart, so that through this method, this interference can be eliminated. Thus, after calibration, it will be able to obtain accurate blood pressure. Besides, advantageously, the user can be sit or stand without influencing the measurement result.

Similarly, the optical sensors also can be mounted at different positions of the body. For example, when two electrodes are both carried by wearable structure, it will be able to respectively mount the optical sensor in each wearable structure, so that a PWV (Pulse Wave Velocity) can be obtained by calculating the time difference between two locations, so as to further obtain reference blood pressure through the known calculation formula. For example, it can be one ear-worn structure with one wrist-worn structure, or two ear-worn structures. There is no limitation.

In another embodiment, the device of the present invention also can cooperate with a cuff and a pump to acquire the blood pressure directly, and in this case, the cuff can be used to acquire the continuous pulse variations for performing the above-mentioned analysis of arrhythmia possible event. It is also advantageous.

Furthermore, the wearable ECG measurement device of the present invention is also suitable for being used during exercise. For example, without burden, the user can wear the device of the present invention during exercise, and in the break time, the user can directly perform the measurement to know the influence of exercise on heart. For example, it can be user's one hand touches the electrode, or it also can be implemented to be both electrodes are carried by the wearable structures, for acquiring ECG signals; and alternatively, the optical sensor also can be used to acquire heart beat sequence. Thereby, according to the information provided by the information providing unit, the user can understand his/her physical condition, for example, if the intensity of exercise is sufficient (the heart rate reaches the target), or is there any abnormal condition of the heart, since there is higher possibility to occur arrhythmia during exercise. Accordingly, through the device of the present invention, any heart condition during exercise can be recorded in real time.

In addition, other than the intense exercise, there are also some other situations that might have abnormal heart rate, such as, mountain climbing and taking an airplane, and these situations are also suitable for using the device of the present invention, so as to timely monitor the heart condition.

In the aforesaid, through the wearable structure, the wearable ECG measurement device of the present invention not only is mounted on the user's body but also completes the contact between the electrode and the skin, so as to reduce the force application from the user and thus also the interference of muscle tensions. In particular, when two electrodes are both carried by the wearable structures, the interference of muscle tension can be reduced to minimum. And, no matter adopting which kind of wearable structure described in the present invention, such as, finger-worn, ear-worn, wrist-worn, and/or head-worn structure, all are similar to the general wearable objects in the daily life and not abrupt, so that it is beneficial for daily usage and thus capable of timely recording the physiological signals as needed, such as, when arrhythmia occurs; and/or for knowing the physiological information, such as, the real time HRV analysis results; and/or for performing physiological procedure, such as, the breathing training. Therefore, it is easy to setup and convenient to use, and can be wide range used.

Further, the wearable ECG measurement device of the present invention also provides two operation modes. In the first operation mode, two electrodes are both located on the surface of the device, and in the second operation mode, one of the electrodes is extended from the device via connecting wire. Accordingly, in different using environments and with different operation habits, the user can therefore have selections. In the second operation mode, the extended electrode also provides the possibility to be positioned at different body potions so as to acquire electrocardiograms at different angles. Besides, as the extended electrode is carried by a wearable structure, there will be no need for the user to apply force during operation, which is also advantageous.

## Claims

1. A wearable electrocardiographic measurement device, comprising:
a circuitry, comprising a processor;
an ear-worn structure, mounted on an ear of a user;
a first electrode and a second electrode, wherein the first electrode is located on a surface of the ear-worn structure which contacts the skin of the ear or around the ear while the device is mounted on the ear, and another surface of the ear-worn structure which doesn't contact the skin of the ear or around the ear comprises the second electrode mounted thereon; and
an information providing unit,
wherein
when performing an electrocardiographic measurement, the user wears the ear-worn structure on the ear to contact the first electrode with the skin of the user or around the ear and uses one hand to contact the second electrode, thereby achieving a loop for acquiring electrocardiographic signals.

2. The device as claimed in claim 1, wherein the ear-worn structure is one selected from a group consisting of: ear clip, ear plug, and ear hook.

3. The device as claimed in claim 1, further comprising plural electroencephalographic electrodes, configured to contact the skin of the ear or around the ear via the ear-worn structure, so as to acquire electroencephalographic signals.

4. The device as claimed in claim 3, wherein the first electrode is configured to be one of the electroencephalographic electrodes.

5. The device as claimed in claim 1, wherein the processor is configured to perform an analysis of the electrocardiographic signals for obtaining a time sequence of heart beats, and compare the time sequence of heart beats with an arrhythmia time sequence characteristic, so as to determine an arrhythmia event.

6. The device as claimed in claim 1, further comprising a transmission module, and the information providing unit is configured to transmit information to an external device via the transmission module, so as to provide the information to the user via the external device.

7. The device as claimed in claim 1, further comprising an optical sensor, positioned on the ear or around the ear together with the first electrode via the ear-worn structure, for detecting consecutive pulse variations of the user.

8. The device as claimed in claim 7, wherein the processor is configured to obtain a time sequence of heart beats based on the consecutive pulse variations.

9. The device as claimed in claim 8, wherein the processor is configured to compare the time sequence of heart beats with an arrhythmia time sequence characteristic, so as to determine a possible arrhythmia event.

10. The device as claimed in claim 9, wherein when the possible arrhythmia event is determined, the processor generates a notification for informing the user the occurrence of possible arrhythmia event via the information providing unit, thereby notifying the user to perform the electrocardiographic measurement.

11. The device as claimed in claim 8, wherein the processor is configured to perform a HRV analysis of the time sequence, so as to obtain the information of autonomic nervous system (ANS) activity.

12. The device as claimed in claim 8, wherein the processor is configured to perform an analysis for obtaining respiratory sinus arrhythmia (RSA) information, and based on the RSA information, the processor generates a breathing guiding signal which is provided to the user via the information providing unit in a breathing training section.

13. The device as claimed in claim 7, wherein the processor is configured to obtain a pulse transit time (PTT) based on the consecutive pulse variations and the electrocardiographic signals, so as to calculate referential values of blood pressure.

14. A wearable electrocardiographic measurement device, comprising:
a circuitry, comprising a processor;
a finger-worn structure, mounted on a finger of a user;
a first electrode and a second electrode, wherein the first electrode is located on a surface of the finger-worn structure which contacts the finger while the device is mounted on the finger, and another surface of the finger-worn structure which doesn't contact the finger comprises the second electrode mounted thereon; and
an information providing unit,
wherein
when performing an electrocardiographic measurement, the user wears the finger-worn structure on the finger to contact the first electrode with the skin thereof and contacts the second electrode with a body portion other than the limb of the finger, thereby achieving a loop for acquiring electrocardiographic signals.

15. The device as claimed in claim 14, further comprising a housing for accommodating at least a portion of the circuitry, wherein the housing is configured to connect with the finger-worn structure via a pair of connectors, and is further positioned on a wrist of another upper limb via a wrist-worn structure.

16. The device as claimed in claim 14, wherein the processor is configured to perform an analysis of the electrocardiographic signals for obtaining a time sequence of heart beats, and compare the time sequence of heart beats with an arrhythmia time sequence characteristic, so as to determine an arrhythmia event.

17. The device as claimed in claim 14, further comprising a transmission module, and the information providing unit is configured to transmit information to an external device via the transmission module, so as to provide the information to the user via the external device.

18. The device as claimed in claim 14, further comprising an optical sensor, positioned on the finger together with the first electrode via the finger-worn structure, for detecting consecutive pulse variations of the user.

19. The device as claimed in claim 18, wherein the processor is configured to obtain a time sequence of heart beats based on the consecutive pulse variations.

20. The device as claimed in claim 19, wherein the processor is configured to compare the time sequence of heart beats with an arrhythmia time sequence characteristic, so as to determine a possible arrhythmia event.

21. The device as claimed in claim 20, wherein when the possible arrhythmia event is determined, the processor generates a notification for informing the user the occurrence of possible arrhythmia event via the information providing unit, thereby notifying the user to perform the electrocardiographic measurement.

22. The device as claimed in claim 19, wherein the processor is configured to perform a HRV analysis of the time sequence, so as to obtain the information of autonomic nervous system (ANS) activity.

23. The device as claimed in claim 19, wherein the processor is configured to perform an analysis for obtaining respiratory sinus arrhythmia (RSA) information, and based on the RSA information, the processor generates a breathing guiding signal which is provided to the user via the information providing unit in a breathing training section.

24. The device as claimed in claim 18, wherein the processor is configured to obtain a pulse transmit time (PTT) based on the consecutive pulse variations and the electrocardiographic signals, so as to calculate referential values of blood pressure.

25. A wearable electrocardiographic measurement device, comprising:
a circuitry, comprising a processor;
a finger-worn structure, mounted on a finger of a user;
an ear-worn structure, mounted on an ear of the user;
a first electrode and a second electrode, wherein the first electrode is located on a surface of the device which contacts the skin of the finger while the finger-worn structure is mounted on the finger, and the second electrode is located on another surface of the device which contacts the skin of the ear or around the ear while the ear-worn structure is mounted on the ear; and
an information providing unit,
wherein
when performing an electrocardiographic measurement, the user wears the finger-worn structure on the finger to contact the first electrode with the skin of the finger, and wears the ear-worn structure on the ear to contact the second electrode with the skin of the ear or around the ear, thereby achieving a loop for acquiring electrocardiographic signals.

26. The device as claimed in claim 25, further comprising plural electroencephalographic electrodes, configured to contact the skin of the ear or around the ear via the ear-worn structure, so as to acquire electroencephalographic signals.

27. The device as claimed in claim 26, wherein the second electrode is configured to be one of the electroencephalographic electrodes.

28. The device as claimed in claim 26, further comprising a head-worn structure having at least one of the plural electroencephalographic electrodes mounted thereon.

29. The device as claimed in claim 25, further comprising a transmission module, and the information providing unit is configured to transmit information to an external device via the transmission module, so as to provide the information to the user via the external device.

30. The device as claimed in claim 25, further comprising at least an optical sensor, mounted on at least one of the finger-worn structure and the ear-worn structure, for detecting consecutive pulse variations of the user.

31. The device as claimed in claim 30, wherein the processor is configured to obtain a pulse transit time (PTT) based on the consecutive pulse variations and the electrocardiographic signals, so as to calculate referential values of blood pressure.

32. The device as claimed in claim 30, wherein the finger-worn structure is configured to have one optical sensor mounted thereon together with the first electrode for mounting on the finger, and the ear-worn structure is configured to have another optical sensor mounted thereon together with the second electrode for mounting on the ear or around the ear, for respectively detecting consecutive pulse variations of the user at the finger and at the ear, and the processor is configured to obtain information related to pulse wave velocity (PWV) based on the consecutive pulse variations, so as to calculate referential values of blood pressure.

33. A wearable electrocardiographic measurement device, comprising:
a circuitry, comprising a processor;
a finger-worn structure, mounted on a finger of a upper limb of a user;
a wrist-worn structure, mounted on a wrist of another upper limb of the user;
a first electrode and a second electrode, wherein the first electrode is located on a surface of the device which contacts the skin of the finger while the finger-worn structure is mounted on the finger, and the second electrode is located on another surface of the device which contacts the skin of the ear or around the ear while the ear-worn structure is mounted on the ear; and
an information providing unit,
wherein
when performing an electrocardiographic measurement, the user wears the finger-worn structure on the finger to contact the first electrode with the skin of the finger, and wears the ear-worn structure on the ear to contact the second electrode with the skin of the ear or around the ear, thereby achieving a loop for acquiring electrocardiographic signals.

34. The device as claimed in claim 33, wherein the finger-worn structure is implemented as a finger-sleeve having an indentation for accommodating the finger tip of the finger, and the first electrode is located in the indentation.

35. The device as claimed in claim 33, wherein the finger-worn structure is implemented as a ring structure.

36. The device as claimed in claim 33, further comprising a third electrode, located on a surface of the wrist-worn structure which is not contact the skin of the wrist.

37. The device as claimed in claim 33, wherein the processor is configured to perform an analysis of the electrocardiographic signals for obtaining a time sequence of heart beats, and compare the time sequence of heart beats with an arrhythmia time sequence characteristic, so as to determine an arrhythmia event.

38. The device as claimed in claim 33, further comprising a transmission module, and the information providing unit is configured to transmit information to an external device via the transmission module, so as to provide the information to the user via the external device.

39. The device as claimed in claim 33, further comprising at least an optical sensor, mounted on at least one of the finger-worn structure and the wrist-worn structure, for detecting consecutive pulse variations of the user.

40. The device as claimed in claim 39, wherein the processor is configured to obtain a pulse transit time (PTT) based on the consecutive pulse variations and the electrocardiographic signals, so as to calculate referential values of blood pressure.

41. The device as claimed in claim 39, wherein the finger-worn structure is configured to have one optical sensor mounted thereon together with the first electrode for mounting on the finger, and the wrist-worn structure is configured to have another optical sensor mounted thereon together with the second electrode for mounting on the wrist, for respectively detecting consecutive pulse variations of the user at the finger and at the wrist, and the processor is configured to obtain information related to pulse wave velocity (PWV) based on the consecutive pulse variations, so as to calculate referential values of blood pressure.

42. A wearable physiological monitoring device, comprising:
a circuitry, comprising a processor;
a head-worn structure, mounted on the head of a user;
a first electrode and a second electrode, wherein the first electrode is located on a surface of the device which contacts the skin of the head while the head-worn structure is mount on the head, and another surface of the device which doesn't contact the skin of the head has the second electrode mounted thereon; and
an information providing unit,
wherein
when performing an electrocardiographic measurement, the user wears the head-worn structure on the head to contact the first electrode with the skin of the head and uses one hand to contact the second electrode, thereby achieving a loop for acquiring electrocardiographic signals; and
wherein
the device further comprises plural electroencephalographic electrodes, for acquiring electroencephalographic signals while the device is mounted on the user's head via the head-worn structure.

43. The device as claimed in claim 42, wherein at least one of the first electrode and the second electrode is configured to be one of the plural electroencephalographic electrodes.

44. The device as claimed in claim 42, wherein the second electrode is mounted on at least of a group consisting of: an ear-worn structure, a finger-worn structure, a wrist-worn structure, and an arm-worn structure.

45. The device as claimed in claim 42, wherein one of the plural electroencephalographic electrodes is mounted on an ear-worn structure.

46. The device as claimed in claim 42, wherein the head-worn structure is implemented as at least one of a group consisting of: a belt, a helmet, a head frame, and a glasses.

47. The device as claimed in claim 42, wherein the processor is configured to perform an analysis of the electrocardiographic signals for obtaining a time sequence of heart beats, and compare the time sequence of heart beats with an arrhythmia time sequence characteristic, so as to determine an arrhythmia event.

48. The device as claimed in claim 42, further comprising a transmission module, and the information providing unit is configured to transmit information to an external device via the transmission module, so as to provide the information to the user via the external device.

49. The device as claimed in claim 42, further comprising an optical sensor, mounted on the head-worn structure, for detecting consecutive pulse variations of the user, and the processor is configured to obtain a time sequence of heart beats based on the consecutive pulse variations.

50. The device as claimed in claim 49, wherein the processor is configured to perform an analysis of the time sequence of heart beats for obtaining the information about the user's breathing pattern, so as to perform a synchronization analysis among electroencephalographic signals, respiration, and heat rate.

51. The device as claimed in claim 49, wherein the processor is configured to compare the time sequence of heart beats with an arrhythmia time sequence characteristic, so as to determine a possible arrhythmia event.

52. The device as claimed in claim 51, wherein when the possible arrhythmia event is determined, the processor generates a notification for informing the user the occurrence of possible arrhythmia event via the information providing unit, thereby notifying the user to perform the electrocardiographic measurement.

53. The device as claimed in claim 49, wherein the processor is configured to perform a HRV analysis of the time sequence, so as to obtain the information of autonomic nervous system (ANS) activity.

54. The device as claimed in claim 49, wherein the processor is configured to perform an analysis for obtaining respiratory sinus arrhythmia (RSA) information, and based on the RSA information, the processor generates a breathing guiding signal which is provided to the user via the information providing unit in a breathing training section.

55. A wearable physiological monitoring device, comprising:
a circuitry, comprising a processor;
a first wearable structure, mounted on the head or ear of a user;
a second wearable structure, mounted on the neck or shoulder of the user;
a first electrode and a second electrode, wherein the first electrode is located on a surface of the first wearable structure which contacts the head or ear while mounted thereon, and the second electrode is mounted on a surface of the second wearable structure which contacts the neck or shoulder while mounted thereon; and
an information providing unit,
wherein
when performing an electrocardiographic measurement, the user wears the first wearable structure on the head or ear to contact the first electrode with the skin thereof and wears the second wearable structure on the neck or shoulder to contact the second electrode with the skin thereof, thereby achieving a loop for acquiring electrocardiographic signals; and
wherein
the device further comprises plural electroencephalographic electrodes mounted on the first wearable structure, so as to acquire electroencephalographic signals while the first wearable structure is mounted on the head or ear.

56. The device as claimed in claim 55, wherein the first electrode is implemented to be one of the plural electroencephalographic electrodes.
